# EUROPEAN PATENT SPECIFICATION

(11) **EP 4 504 098 B1**
(45) Date of publication and mention of the grant of the patent: **20.08.2025**
(21) Application number: 24709071.5
(22) Date of filing: 07.03.2024
(51) Int. Cl.: A61F 2/12

(54) **BREAST IMPLANT**
BRUSTIMPLANTAT
IMPLANT MAMMAIRE

(30) Priority: 09.03.2023 EP 23160879
(43) Date of publication of application: 12.02.2025
(73) Proprietor: HELMo Link, 4000 Liège (BE); Université de Liège, 4000 Liège (BE)
(72) Inventor: OPRENYESZK, Frédéric, 4053 Embourg (BE); PIERRE, Julien, 4140 Sprimont (BE); QUINTING, Birgit, 6900 Roy (BE); STORDEUR, Philippe, 4520 Vinalmont (BE); NOLENS, Grégory, 4680 Oupeye (BE); NIZET, Christophe, 4000 Liège (BE)
(74) Representative: Gevers Patents
(86) International application number: PCT/EP2024/056014
(87) International publication number: WO 2024/184459

(56) References cited:
- WO-A2-2021/188975
- US-A- 4 650 487
- US-A1- 2021 236 266

## Description

### Field of the invention

The present invention relates to a breast implant.

### Background of the invention

Breast implants are typically used to increase the size of the breast or to replace a removed breast or part of breast. Some known breast implants are designed to be progressively absorbed by the body while being replaced by body tissues. Document US 2021/0236266 A1 describes tissue engineering devices and methods employing scaffolds made of absorbable material for use in the human body for tissue genesis and regenerative and cellular medicine including breast reconstruction.

With gravity, a breast tends to fall, sometimes giving rise to a breast ptosis, where the areola is too low and the gland goes below the level of the submammary fold. A breast ptosis may happen in a breast without implant but also with a breast including an implant, especially when the implant is absorbed by the body.

### Summary of the invention

An object of the invention is to provide a breast implant decreasing the risk of breast ptosis.

According to this first aspect, the invention provides a breast implant comprising:
- a first part extending, from a basis, along an axis and comprising a first scaffold, the first scaffold being absorbable and being made of first patterns of a first shape,
- a second part radially surrounding the first part and comprising a second scaffold, the second scaffold being absorbable and being made of second patterns of a second shape, the second shape being different from the first shape,
wherein the first part is more rigid than the second part, and wherein the first part is less absorbable than the second part such that a longer time is required to dissolve the first part in a mammal body than to dissolve the second part in same mammal body.

The flexibility of the second part is advantageous during the positioning of the implant, while the rigidity of the first part decreases the risk of breast ptosis.

Since the first and the second scaffolds are absorbable, they progressively dissolve in the body. They can be said bio-absorbable. With the implant according to the invention, the first part, i.e., the axial part, remains longer than the second part, which further decreases the long term risk of breast ptosis.

The higher rigidity and the lower absorbability of the first part with respect to the second part may be due to several characteristics of the first part and the second part, especially of the first scaffold and the second scaffold.

The higher rigidity of the first part with respect to the second part comes, at least in part, from the difference in shape between the first and the second patterns. Indeed, the geometry of the pattern influences the bending of the scaffold. The lower absorbability of the first part with respect to the second part may also come, at least in part, from the difference in shape between the first and the second patterns. For example, both patterns may comprise polygons, the second pattern polygons having fewer sides than the first pattern polygons.

The first part is preferably extending further along the axis than in any direction perpendicular.

In average, the first part is denser than the second part. The second part surrounds the first part at 360°.

The first part is more rigid than the second part at least in the radial directions: a higher force is required to bend a portion of the first part than to bend an equivalent portion of the second part.

The first part is less absorbable than the second part: a longer time is required to dissolve the first part in a mammal body than to dissolve the second part in same body. In an embodiment, the first part is configured for taking 3 month, preferably 6 months, more preferably 9 months longer than the second part to dissolve in a mammal body. In the present application, the mammal body is preferably a human body such as a female human body.

The basis is preferably planar. After implantation, the basis is expected to be vertical or approximately vertical when the patient is standing.

The external surface of the breast implant comprises, preferably consists in, the basis and a curved external surface. The basis and the curved external surface are porous.

The implant comprises a top, located on the curved external surface. The top is the point of the curved external surface that is the furthest from the base. The top preferably corresponds to the areola of the breast.

In the frame of the present document, an axial direction is a direction parallel to the axis and a radial direction is perpendicular to the axis.

The implant is elastically deformable in a direction perpendicular to the axis.

The first and the second scaffolds are suitable for colonization by the vascular network with fat cells (differentiated cells) and their precursors (undifferentiated cells).

The first and the second scaffolds are three-dimensional structures. The first and the second scaffolds are preferably homogeneous. They may be anisotropic. The first and the second patterns are preferably three-dimensional structures.

The first scaffold is preferably a repetition of the first patterns, preferably in the three dimensions. The second scaffold is preferably a repetition of the second patterns, preferably in the three dimensions.

In an embodiment, the first and/or second pattern can be a square, a rectangle, a triangle, a polygon, a cube, a tetrahedron.

A pattern is preferably a mesh. The first (respectively second) pattern may be considered, in an embodiment of the invention, as the smallest building unit that repeats, possibly with a rotation, in three dimensions to form the first (respectively second) scaffold. A scaffold is preferably a three-dimensional meshing. A scaffold is porous.

The first scaffold is more rigid and less absorbable than the second scaffold.

If the first part comprises two or more scaffolds and/or the second part comprises two or more scaffolds, at least one of the scaffold(s) of the first part is more rigid and less absorbable than at least one of the scaffold(s) of the second part.

In an embodiment wherein the first part comprises two or more scaffolds and/or the second part comprises two or more scaffolds, it is possible that any of the scaffold(s) of the first part is more rigid and less absorbable than any of the scaffold(s) of the second part. All scaffolds are absorbable.

The first scaffold comprises a plurality of first bars and/or first plates and the second scaffold comprises a plurality of second bars. Preferably, the first scaffold consists in a plurality of first bars and/or first plates, and/or the second scaffold consists in a plurality of second bars. The first bars and/or first plates preferably form the first patterns. The second bars preferably form the second patterns. The bars are preferably straight. The plates are preferably planar. Some or all of the plates may be parallel to the axis. Some or all of the plates may be perpendicular to the axis.

The first bars and/or first plates are preferably distributed in at least three directions in order to form a three-dimensional grid. The second bars are preferably distributed in at least three directions in order to form a three-dimensional grid. In an embodiment of the invention, the second bars are distributed in only three directions. The bars and/or plates contact each other at crossings where they stick to each other.

The first and the second scaffolds are in polymer. The Young modulus of the material(s) of the first scaffold is preferably between 50 and 5000 MPa, preferably between 100 and 5000 MPa. The Young modulus of the material(s) of the second scaffold is preferably between 50 and 5000 MPa, preferably between 100 and 5000 MPa.

The axis is preferably the anteroposterior axis. The axis is preferably perpendicular to the basis. The axis extends between the basis and the curved external surface. The axis preferably extends between the basis and the top. The axis is not necessarily a symmetry axis of the breast implant.

The first end of the first part is preferably located on the basis. It preferably includes the center of the basis. The second end of the first part is preferably on the curved external surface. The second end of the first part preferably extends to the top.

The limit between the first part and the second part is a transition interface.

In an embodiment, the second scaffold comprises a plurality of second bars and
- the first scaffold comprises a plurality of first bars comprising bars in more directions than the plurality of second bars, and/or
- the first scaffold comprises a plurality of first plates.

In other words, the first scaffold is distributed in more directions than the second scaffold. This increases the rigidity of the first scaffold with respect to the second scaffold. For example, the first patterns may comprise hexagonal sides (with bars in three directions) or plates parallel to the axis, while the second patterns may comprise rhombic sides (with bars in two directions).

In an embodiment, the first scaffold comprises a plurality of first bars and/or first plates and the second scaffold comprises a plurality of second bars, a thickness of the plurality of first bars and/or the thickness of the plurality of first plates being at least 1,3 times a thickness of the second bars.

The first bars and/or first plates are preferably at least twice thicker than the second bars i.e., the thickness of the first bars and/or first plates are preferably at least 2 times the thickness of the second bars. This increases the absorbability of the second scaffold with respect to the first scaffold.

In an embodiment, the first part comprises a plurality of scaffolds, said plurality of scaffolds comprising at least once the first scaffold.

The first scaffold, with its first patterns of the first shape, may be repeated several times within the first part, the different instances of the first scaffold being spaced from each other. A volume of an instance of the first scaffold may be defined as the zone of space occupied by i.e., filled by the different instances of the first scaffold. For example, the sum of the volumes of all the instances of the first scaffold is at least 70% of a volume of the first part.

In an embodiment, a volume of the first scaffold, or a sum of volumes of a plurality of scaffolds comprising at least once the first scaffold, occupies at least 70% of a volume of the first part. Preferably, the volume of the first scaffold, or the sum of the volumes of the plurality of scaffolds comprising at least once the first scaffold, occupies 100% of the volume of the first part. This means that at least 70%, and preferably 100%, of the volume of the first part is occupied by the first scaffold or by the plurality of scaffolds comprising at least once the first scaffold. Preferably, said plurality of scaffolds comprising at least once the first scaffold are all based on patterns of the first shape.

In the present document, the volume of the first part includes the material within the first part and the empty space within the first part. Similarly, the volume of the first scaffold includes the material within the first scaffold and the empty space within the first scaffold. Similarly, the volume of the first pattern includes the material within the first pattern and the empty space within the first pattern.

In the present document, the volume of the second part includes the material within the second part and the empty space within the second part. Similarly, the volume of the second scaffold includes the material within the second scaffold and the empty space within the second scaffold. Similarly, the volume of the second pattern or any other element includes the material within the second pattern or any other element, respectively and the empty space within the second pattern or any other element, respectively.

Before implanting the breast implant, the volume of an instance of the first scaffold thus comprises absorbable material of the scaffold and empty space filled for instance by air. For example, a volume filling rate of the volume of the instance of the first scaffold by the absorbable material is preferably comprised between 5% and 40%, typically between 10% and 25%.

In an embodiment, a volume of the second scaffold, or a sum of volumes of a plurality of scaffolds comprising at least once the second scaffold, occupies at least 70% of a volume of the second part. Preferably, the volume of the second scaffold, or the sum of the volumes of the plurality of scaffolds comprising at least once the second scaffold, occupies 100% of the volume of the second part. This means that at least 70%, and preferably 100%, of the volume of the second part is occupied by the volume of the second scaffold or by the sum of the volumes of the plurality of scaffolds comprising at least once the second scaffold.

In an embodiment, the first part has a horizontal radial extension that is between 10% and 65% of the horizontal extension of the basis. The radial extensions are measured along the basis.

In an embodiment, the second part has a lower density than the first part. In other words, the ratio between the mass and the occupied volume is higher for the first part than for the second part. This helps in decreasing the absorbability of the first part with respect to the second part.

In an embodiment, the volume of the first part is comprised between 5 cm³ and 300 cm³, preferably between 15 cm³ and 165 cm³. In an embodiment, the volume of the second part is comprised between 100 cm³ and 700 m³, preferably between 200 cm³ and 600 cm³.

In an embodiment, a ratio of the volume of the first part to the volume of the second part is comprised between 2.5 % and 50 %, preferably between 15 % and 24 %.

Preferably, the second scaffold has a lower density than the first scaffold.

In an embodiment, first patterns have a lower volume than second patterns. Preferably, each of the first patterns have a lower volume than each of the second patterns. Smaller patterns help in increasing the rigidity and decreasing the absorbability.

In an embodiment, the first pattern has openings and the second pattern has openings such that the openings of the first pattern are smaller than the openings of the second pattern. The openings may for example be in a vertical plan parallel to the basis.

In an embodiment, the first patterns have a dimension between 1 mm et 8 mm. A dimension of the first patterns, which is preferably the distance between two adjacent parallel first bars and/or first plates, is preferably between 2 and 4 mm. Said dimension is preferably measured vertically.

In an embodiment, the second patterns have a dimension between 2 mm and 10 mm. A dimension of the second patterns, which is preferably the distance between two adjacent parallel second bars, is preferably between 4 and 6 mm. Said dimension is preferably measured vertically.

The dimension of the first pattern is preferably lower than the dimension of the second pattern.

In an embodiment, the first part is removable with respect to the second part. In another embodiment of the invention, the first part is not removable with respect to the second part.

In an embodiment, the breast implant is an assembly of the first part and the second part. This means that the breast implant is obtained by assembling the first part and the second part after producing them separately.

The disclosure relates also to a process to produce a breast implant using additive manufacturing to produce the first scaffold and the second scaffold. The first and the second scaffold may be printed as separated pieces, or, preferably, may be printed as a single piece. The breast implant is preferably printed as a single piece.

In an example, the additive manufacturing involves a deposition of a fused filament.

### Brief description of the figures

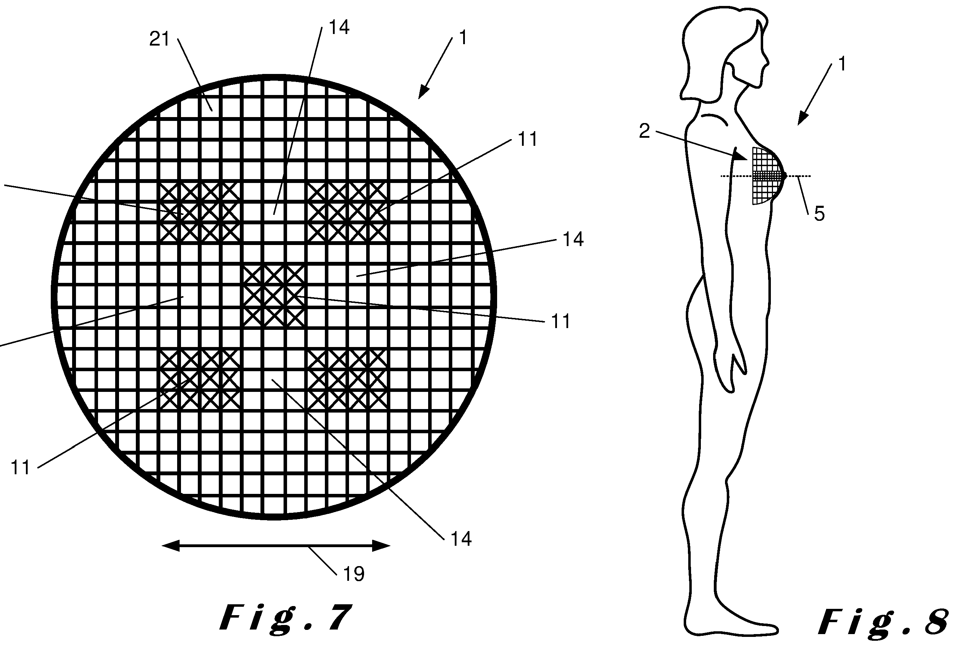

For a better understanding of the present invention, reference will now be made, by way of example, to the accompanying drawings. In the description of the drawing, the implant is supposed to be within a standing patient, as illustrated on Figure 8.
- Figure 1 is a low-angle view illustrating an implant according to an embodiment of the invention from a point of view facing the implant holder and being slightly below the implant;
- Figure 2a shows the back side of the implant in an exemplary first embodiment;
- Figure 2b shows the back side of the implant in an exemplary second embodiment;
- Figure 3a shows the front side of the implant in the exemplary first embodiment;
- Figure 3b shows the front side of the implant in the exemplary second embodiment;
- Figure 4a shows a vertical cross sectional view along the cutting plane IVa shown in Figures 2a and 3a of the implant in the exemplary first embodiment;
- Figure 4b shows a vertical cross sectional view along the cutting plane IVb shown in Figures 2b and 3b of the implant in the exemplary second embodiment;
- Figures 5a and 5b illustrate two possible embodiments of a first pattern;
- Figures 6a and 6b illustrate two possible embodiments of a second pattern;
- Figure 7 shows the back side of the implant in a variation of the first embodiment;
- Figure 8 shows a standing patient implanted with a breast implant.

### Description of the invention

The present invention will be described with respect to particular embodiments and with reference to certain drawings but the invention is not limited thereto. The described functions are not limited by the described structures. The drawings described are only schematic and are non-limiting. In the drawings, the size of some of the elements may be exaggerated and not drawn on scale for illustrative purposes.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present invention, the only enumerated components of the device are A and B, and further the claim should be interpreted as including equivalents of those components.

On the figures, identical or analogous elements may be referred by a same number.

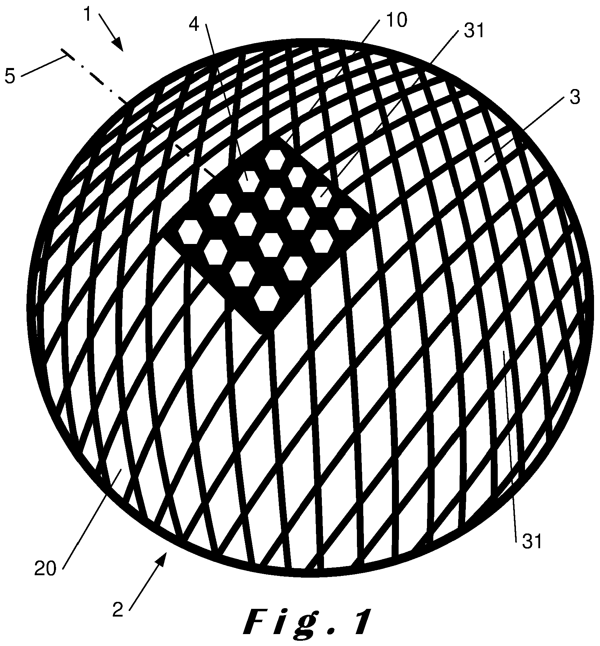

Figure 1 illustrates a breast implant 1 according to an embodiment of the invention. The breast implant 1 has a basis 2 and an external curved surface 3, with a top 4 opposed to the basis 2. The breast implant 1 comprises a first part 10 extending along an axis 5, and a second part 20 located around the first part 10. The basis 2 is partially included in the first part 10 and partially included in the second part 20. The first part 10 and the second part 20 extend from the basis 2 towards the external curved surface 3.

The basis 2 is expected to be placed to be vertical on a standing patient (as illustrated on Figure 8). The axis 5 is preferably expected to be horizontal towards the front, i.e. perpendicular to the basis.

The curved external surface 3 may be a hemispheric dome (first embodiment of the invention) or an asymmetric dome (second embodiment of the invention), with the top 4 shifted down (corresponding to the shape of a breast hanging down). Other shapes are possible without departing from the frame of the invention.

In the first embodiment of the invention, the basis 2 is preferably circular or have the shape of a polygon with at least 10 sides. In the second embodiment of the invention, the basis 2 is preferably an oval.

All combination of features hereby described as being part of the first or the second embodiments of the invention are possible within the frame of the present invention.

The curved external surface 3 is preferably a meshing, preferably with holes 31 opening in all directions forward. The holes 31 preferably have an opening of at least 2 mm by 2 mm, preferably of at least 1 by 1 mm, in the first part 10 and in the second part 20. Such a minimum dimension is preferred in order to make possible an injection from all direction forward.

The first part 10 is more rigid and less absorbable than the second part 20. Preferably, the second part 20 has a lower density than the first part 10.

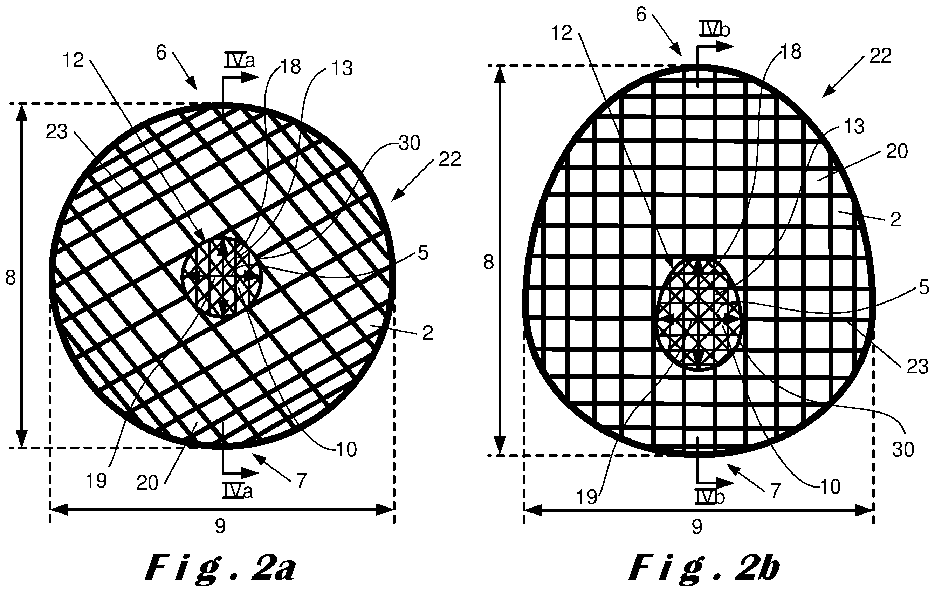

Figure 2a illustrates a possible variation of the basis 2 in the first embodiment of the invention. Figure 2b illustrates a possible variation of the basis 2 in the second embodiment of the invention.

The first part 10 comprises a first scaffold 11, which is preferably located directly around the axis 5. The first scaffold 11 is made of first patterns 12. The first part 10 may comprise other elements than the first scaffold 11, for example it may comprise other scaffolds or absorbable non-porous parts. At least, preferably 70% of the volume of the first part 10 is taken by the first scaffold 11 or a plurality of scaffolds comprising at least once the first scaffold 11.

The second part 20 comprises a second scaffold 21, which preferably radially surrounds the first part 10. The second scaffold 21 is made of second patterns 22. The second part 20 may comprise other elements than the second scaffold 21, for example it may comprise other scaffolds or absorbable non-porous parts. At least, preferably 70% of the volume of the second part 20 is taken by the second scaffold 21 or a plurality of scaffolds comprising at least once the second scaffold 21.

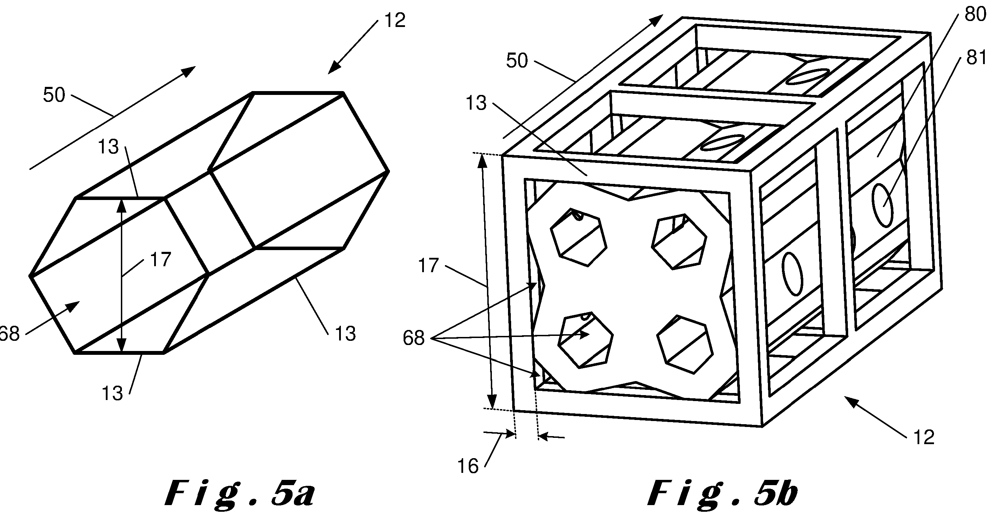

The first scaffold 11 preferably comprises a plurality of first bars 13 and/or first plates 80 (visible at Figure 5b) and the second scaffold 21 comprises a plurality of second bars 23. The first bars 13 and/or first plates 80 preferably have a thickness 16 (illustrated at Figure 5b ) that is at least 1,3 times the thickness of the second bars 23. The first bars 13 and/or first plates 80 more preferably have a thickness 16 (illustrated at Figure 5b ) that is at least between 1,7 and 2,3 times the thickness of the second bars 23.

The first bars 13 and/or first plates 80 may have a thickness 16 (illustrated at Figure 5b) between 1,0 and 4,0 mm, preferably between 2,0 and 3,0 mm. The first scaffold 11 may comprise first bars 13 of several thicknesses 16. Two first bars 13 may be next to each other to form a thicker first bar.

The second bars 23 may have a thickness between 0,3 and 2,5 mm, preferably between 1,0 and 1,2 mm. The second scaffold 21 may comprise second bars 23 of several thicknesses, for example 0,3 and 1,2 mm. Two second bars 23 of 1,2 mm may be next to each other to form a second bar 23 of 2,4 mm by 1,2 mm.

The first patterns 12 have a first shape and the second patterns 22 have a second shape, which is different from the first shape. The second patterns 22 are not the same as the first patterns with a different size or with a different size of the bars 13, 23: the second shape in itself is different than the first shape. For example, as illustrated in Figures 2a and 2b, the number of directions of the first bars 13 within the first pattern 12 may be higher than the number of directions of the second bars 23 within the second pattern 22.

The implant 1 has a higher tip 6, located at the higher junction between the basis 2 and the curved external surface 3. The implant 1 has a lower tip 7, located at the lower junction between the basis 2 and the curved external surface 3. The higher tip 6 and the lower tip 7 are preferably vertically aligned. The vertical extension of the basis 8, between the lower tip 7 and the higher tip 6, may be lower, equal or higher than the horizontal extension of the basis 9, perpendicular to the vertical extension 8. The vertical extension of the first part 18, measured along the basis 2, may be lower, equal or higher than the horizontal extension of the first part 19, measured along the basis 2 and perpendicular to the vertical extension 18. The horizontal extension of the basis 9 is preferably 1,5 to 3,5 times the horizontal extension of the first part 19.

The vertical extension of the basis 8 is preferably in the range between 8 cm and 16 cm. The horizontal extension of the basis 9 is preferably in the range between 8 cm and 16 cm.

The interface between the first part 10 and the second part 20 is a transition interface 30. The transition interface 30 is preferably a cylinder, for example a cylinder with a square basis, a rectangular basis or a circular basis. However, the transition interface 30 may have another shape, for example a conical shape, without departing from the scope of the present invention. The first part 10 may be, or not, removable with respect to the second part 20. The first part 10 may be engaged in a hole of the second part 20. For example, the breast implant may be an assembly of the first and second parts. In this case, the first part 10 and the second part 20 may be produced separately and assembled afterwards.

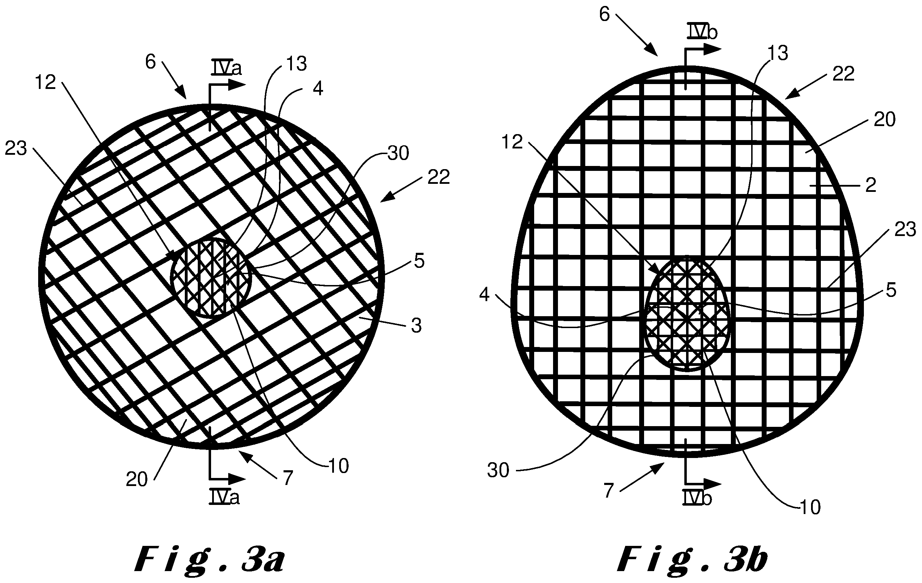

Figure 3a illustrates a possible variation of the curved external surface 3 in the first embodiment of the invention. Figure 3b illustrates a possible variation of the curved external surface 3 in the second embodiment of the invention.

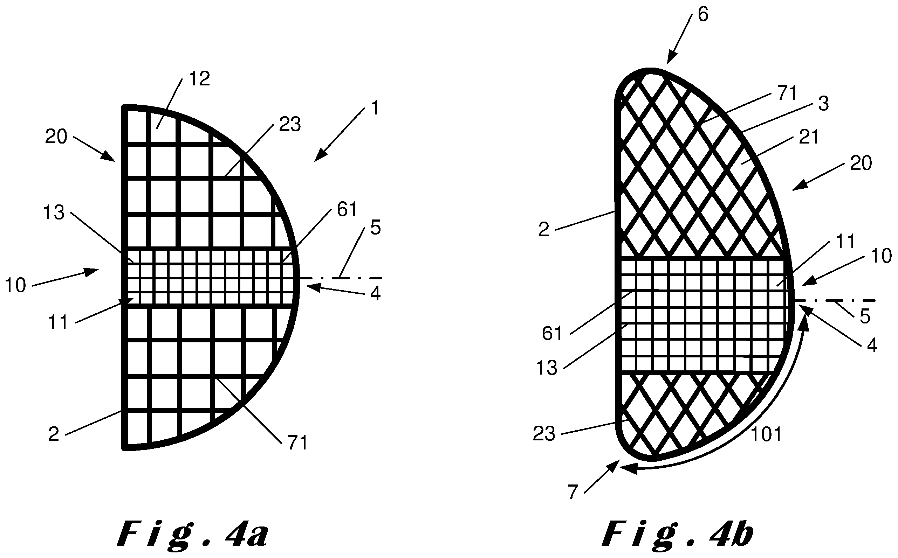

Figure 4a illustrates a possible variation of the implant 1 in the first embodiment of the invention. Figure 4b illustrates a possible variation of the implant 1 in the second embodiment of the invention. These figures illustrate in particular crossings 61 between first bars 13 and crossings 71 between second bars 23. In the invention, it is preferred that the first bars 13 stick to each other at the crossings 61, and the second bars 23 stick to each other at the crossings 71.

The distance between the basis 2 and the top 4 is preferably in the range between 3 and 9 cm. The arc distance 101 (Figure 4b) in the second embodiment is preferably in the range between 4 and 10 cm.

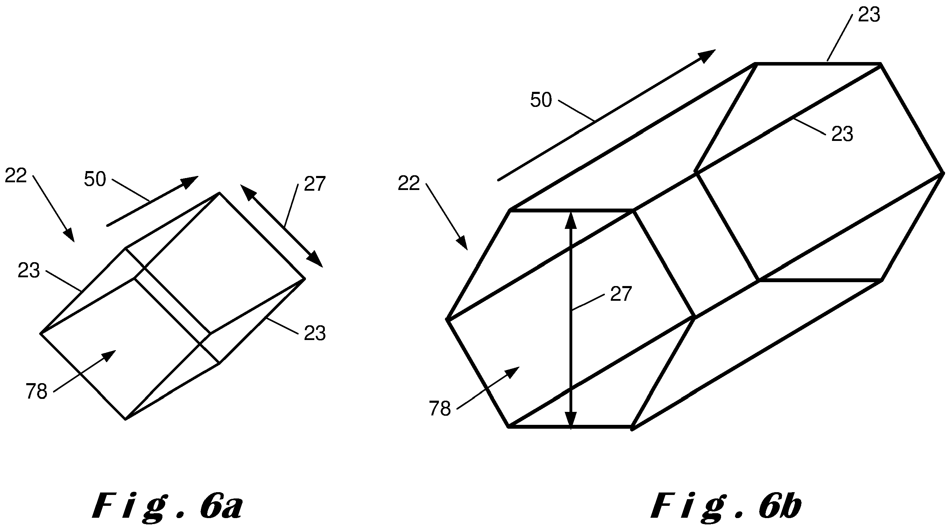

Figures 5a and 5b illustrate two exemplary embodiments of the first pattern 12. Figures 6a and 6b illustrate two exemplary embodiments of the second pattern 22. Arrow 50 indicates a direction parallel to the axis 5. In Figures 5a, 6a, and 6b, the bars 13, 23 are illustrated as lines for the sake of illustration, but they have a thickness of course. The thickness 16 of a first bar 13 is illustrated at Figure 5b. The first plates 80, which are preferably perforated with holes 81, are also illustrated at Figure 5b.

The first pattern 12 may have different shapes. It may include a hexagon (Figure 5a) or a star (Figure 5b), preferably in a plan perpendicular to the axis 5. The second pattern 22 may have different shapes. It may include a rhombus (Figure 6a) or a hexagon (Figure 6b), preferably in a plan perpendicular to the axis 5.

The first pattern 12 has a dimension 17, which is the distance between two parallel bars, for example taken vertically, that is preferably between 2 mm and 4 mm. The second pattern 22 has a dimension 27, which is the distance between two parallel bars, for example taken vertically, that is preferably between 4 mm and 6 mm.

The first pattern 12 has preferably a lower volume than the second pattern 22.

The first pattern 12 has preferably an opening 68 in a plan perpendicular to the axis 5. The second pattern 22 has preferably an opening 78 in a plan perpendicular to the axis 5. The area of the opening 68 of the first pattern 12 is preferably lower than the area of the opening 78 of the second pattern 22.

Since the shape of the first 12 and second 22 patterns are different, the first pattern 12 of Figure 5a may combine with the second pattern 22 of Figure 6a, but not with the second pattern of Figure 6b. The first pattern 12 of Figure 5b may combine with the second pattern 22 of Figure 6a or Figure 6b.

Figure 7 illustrates a possible variation of an implant 1 according to the invention. The first part 10 comprises a plurality of scaffolds. The plurality of scaffolds comprises several times the first scaffold 11 and several times a third scaffold 14. The third scaffold 14 may be identical to the second scaffold 21.

Figure 8 illustrates the position of the breast implant 1 within a patient body.

The first and the second parts may be in the same material or in different materials. The first and/or second scaffold may be for example in any or in several of the following materials: Polydioxanone; Poly(caprolactone) and Poly(L-lactide-co-glycolide); Poly(L-lactide); Poly(L-lactide-co-D, L-Lactide); Poly(D,L-lactide); Poly(L-lactide-co-ε-caprolactone) in various ratios (for example 70 :30); PEG with Poly(L-lactide) or Poly(L-lactide-co-D, L-lactide ; Poly(glycolide-co-trimethylene carbonate); Poly(glycolide); Poly(glycolide-co-L-lactide).

The implant 1, or at least one of the first 11 and second 12 scaffolds, is preferably produced by additive manufacturing, for example by a process of deposition of a fused filament.

In other words, the disclosure relates to a breast implant 1 and to a process to produce at least part of it by additive manufacturing. The breast implant 1 includes a first part 10, more rigid, along an axis 5, and a second part 20, more resorbable, around the first part 10. The implant 1 is bio-absorbable and elastically deformable in a direction perpendicular to the axis 5. The implant 1 is suitable for colonization by the vascular network with fat cells (differentiated cells) and their precursors (undifferentiated cells).

Although the present invention has been described above with respect to particular embodiments, it will readily be appreciated that other embodiments are also possible. In particular, examples are not limiting and that equivalents could be envisaged without departing from the essence of the invention. The functions are not limited by the structures provided in the present document.

The first part is less absorbable than the second part, such that the first part takes a longer time than the second part to be absorbed by a mammal body. For example, this characteristic can be verified by a subcutaneous implantation test in a mammal body. In such a test, a volume V1 filled by a first scaffold of the first part (the first scaffold being made of first patterns of the first shape) and a volume V2 identical to V1 and filled by a second scaffold of the second part (the second scaffold being made of second patterns of the second shape) are implanted in a same mammal body, preferably at a level of the breast of the mammal body. Then, the times required for the absorption of the volumes V1 and V2 by the mammal body are monitored and compared.

## Claims

1. Breast implant (1) comprising:
∘ a first part (10) extending, from a basis (2), along an axis (5) and comprising a first scaffold (11), the first scaffold (11) being absorbable and being made of first patterns (12) of a first shape,
∘ a second part (20) radially surrounding the first part (10) and comprising a second scaffold (21), the second scaffold (21) being absorbable and being made of second patterns (22) of a second shape, the second shape being different from the first shape,
wherein the first part (10) is more rigid than the second part (20), and **characterized in that** the first part (10) is less absorbable than the second part (20) such that a longer time is required to dissolve the first part (10) in a mammal body than to dissolve the second part (20) in same mammal body.

2. Breast implant according to claim 1, wherein the second scaffold (21) comprises a plurality of second bars (23) and
• the first scaffold (11) comprises a plurality of first bars (13) comprising bars in more directions than the plurality of second bars (23), and/or
• the first scaffold (11) comprises a plurality of first plates (80).

3. Breast implant according to any of the preceding claims, wherein the first scaffold (11) comprises a plurality of first bars (13) and/or first plates (80) and the second scaffold (21) comprises a plurality of second bars (23), a thickness (16) of the first bars (13) and/or a thickness (16) of the first plates (80) being at least 1,3 times a thickness of the second bars (23).

4. Breast implant according to any of the preceding claims, wherein the first part (10) comprises a plurality of scaffolds (11, 14), said plurality of scaffolds comprising at least once the first scaffold (11).

5. Breast implant according to any of the preceding claims, wherein a volume of the first scaffold (11), or a sum of volumes of a plurality of scaffolds comprising at least once the first scaffold (11), occupies at least 70% of a volume of the first part (20).

6. Breast implant according to any of the preceding claims, wherein a volume of the second scaffold (21), or a sum of volumes of a plurality of scaffolds comprising at least once the second scaffold (21), occupies at least 70% of a volume of the second part (20).

7. Breast implant according to any of the preceding claims, wherein the first part (10) has a horizontal radial extension (19) that is between 10% and 65% of the horizontal extension (9) of the basis (2).

8. Breast implant according to any of the preceding claims, wherein the second part (20) has a lower density than the first part (10).

9. Breast implant according to any of the preceding claims, wherein a volume of each first pattern (12) is lower than a volume of each second pattern (22).

10. Breast implant according to any of the preceding claims, wherein the first pattern (12) has openings (68) and the second pattern (22) has openings (78) such that the openings (68) of the first pattern (12) are smaller than the openings (78) of the second pattern (22).

11. Breast implant according to any of the preceding claims, wherein the first patterns (12) have a dimension (17) between 1 mm et 8 mm.

12. Breast implant according to any of the preceding claims, wherein the second patterns (22) have a dimension (27) between 2 mm et 10 mm.

13. Breast implant according to any of the preceding claims, wherein the breast implant is an assembly of the first part (10) and the second part (20).

## Patentansprüche

1. Brustimplantat (1), umfassend:
∘ einen ersten Teil (10), der sich von einer Basis (2) entlang einer Achse (5) erstreckt und ein erstes Gerüst (11) umfasst, wobei das erste Gerüst (11) resorbierbar ist und aus ersten Mustern (12) einer ersten Form gefertigt ist,
∘ einen zweiten Teil (20), der den ersten Teil (10) radial umgibt und ein zweites Gerüst (21) umfasst, wobei das zweite Gerüst (21) resorbierbar ist und aus zweiten Mustern (22) einer zweiten Form gefertigt ist, wobei sich die zweite Form von der ersten Form unterscheidet,
wobei der erste Teil (10) steifer ist als der zweite Teil (20), und
**dadurch gekennzeichnet, dass** der erste Teil (10) weniger resorbierbar ist als der zweite Teil (20), sodass eine längere Zeit erforderlich ist, um den ersten Teil (10) in einem Körper eines Säugetiers aufzulösen, als den zweiten Teil (20) in einem gleichen Körper eines Säugetiers aufzulösen.

2. Brustimplantat nach Anspruch 1, wobei das zweite Gerüst (21) eine Vielzahl von zweiten Stäben (23) umfasst, und
• das erste Gerüst (11) eine Vielzahl von ersten Stäben (13) umfasst, die Stäbe in mehr Richtungen umfassen als die Vielzahl von zweiten Stäben (23), und/oder
• das erste Gerüst (11) eine Vielzahl von ersten Platten (80) umfasst.

3. Brustimplantat nach einem der vorstehenden Ansprüche, wobei das erste Gerüst (11 ) eine Vielzahl von ersten Stäben (13) und/oder ersten Platten (80) umfasst, und das zweite Gerüst (21) eine Vielzahl von zweiten Stäben (23) umfasst, wobei eine Dicke (16) der ersten Stäbe (13) und/oder eine Dicke (16) der ersten Platten (80) mindestens das 1,3-Fache einer Dicke der zweiten Stäbe (23) beträgt.

4. Brustimplantat nach einem der vorstehenden Ansprüche, wobei der erste Teil (10) eine Vielzahl von Gerüsten (11, 14) umfasst, wobei die Vielzahl von Gerüsten mindestens einmal das erste Gerüst (11) umfasst.

5. Brustimplantat nach einem der vorstehenden Ansprüche, wobei ein Volumen des ersten Gerüsts (11) oder eine Summe von Volumina einer Vielzahl von Gerüsten, die mindestens einmal das erste Gerüst (11) umfasst, mindestens 70% eines Volumens des ersten Teils (20) einnimmt.

6. Brustimplantat nach einem der vorstehenden Ansprüche, wobei ein Volumen des zweiten Gerüsts (21) oder eine Summe von Volumina einer Vielzahl von Gerüsten, die mindestens einmal das zweite Gerüst (21) umfasst, mindestens 70% eines Volumens des zweiten Teils (20) einnimmt.

7. Brustimplantat nach einem der vorstehenden Ansprüche, wobei der erste Teil (10) eine horizontale radiale Erstreckung (19) aufweist, die zwischen 10% und 65% der horizontalen Erstreckung (9) der Basis (2) beträgt.

8. Brustimplantat nach einem der vorstehenden Ansprüche, wobei der zweite Teil (20) eine geringere Dichte aufweist als der erste Teil (10).

9. Brustimplantat nach einem der vorstehenden Ansprüche, wobei ein Volumen jedes ersten Musters (12) geringer ist als ein Volumen jedes zweiten Musters (22).

10. Brustimplantat nach einem der vorstehenden Ansprüche, wobei das erste Muster (12) Öffnungen (68) aufweist und das zweite Muster (22) Öffnungen (78) aufweist, sodass die Öffnungen (68) des ersten Musters (12) kleiner sind als die Öffnungen (78) des zweiten Musters (22).

11. Brustimplantat nach einem der vorstehenden Ansprüche, wobei die ersten Muster (12) eine Abmessung (17) zwischen 1 mm und 8 mm aufweisen.

12. Brustimplantat nach einem der vorstehenden Ansprüche, wobei die zweiten Muster (22) eine Abmessung (27) zwischen 2 mm und 10 mm aufweisen.

13. Brustimplantat nach einem der vorstehenden Ansprüche, wobei das Brustimplantat eine Anordnung aus dem ersten Teil (10) und dem zweiten Teil (20) ist.

## Revendications

1. Implant mammaire (1) comprenant :
∘ une première partie (10) s'étendant, à partir d'une base (2), le long d'un axe (5) et comprenant un premier échafaudage (11), le premier échafaudage (11) étant résorbable et étant fait de premiers motifs (12) d'une première forme,
∘ une seconde partie (20) entourant radialement la première partie (10) et comprenant un second échafaudage (21), le second échafaudage (21) étant résorbable et étant fait de seconds motifs (22) d'une seconde forme, la seconde forme étant différente de la première forme,
dans lequel la première partie (10) est plus rigide que la seconde partie (20), et
**caractérisé en ce que** la première partie (10) est moins résorbable que la seconde partie (20) de sorte qu'un temps plus long est nécessaire pour dissoudre la première partie (10) dans un corps de mammifère que pour dissoudre la seconde partie (20) dans le même corps de mammifère.

2. Implant mammaire selon la revendication 1, dans lequel le second échafaudage (21) comprend une pluralité de secondes barres (23) et
• le premier échafaudage (11) comprend une pluralité de premières barres (13) comprenant des barres dans plus de directions que la pluralité de secondes barres (23), et/ou
• le premier échafaudage (11) comprend une pluralité de premières plaques (80).

3. Implant mammaire selon l'une quelconque des revendications précédentes, dans lequel le premier échafaudage (11) comprend une pluralité de premières barres (13) et/ou de premières plaques (80) et le second échafaudage (21) comprend une pluralité de secondes barres (23), une épaisseur (16) des premières barres (13) et/ou une épaisseur (16) des premières plaques (80) étant d'au moins 1,3 fois une épaisseur des secondes barres (23).

4. Implant mammaire selon l'une quelconque des revendications précédentes, dans lequel la première partie (10) comprend une pluralité d'échafaudages (11, 14), ladite pluralité d'échafaudages comprenant au moins une fois le premier échafaudage (11).

5. Implant mammaire selon l'une quelconque des revendications précédentes, dans lequel un volume du premier échafaudage (11), ou une somme de volumes d'une pluralité d'échafaudages comprenant au moins une fois le premier échafaudage (11), occupe au moins 70 % d'un volume de la première partie (20).

6. Implant mammaire selon l'une quelconque des revendications précédentes, dans lequel un volume du second échafaudage (21), ou une somme de volumes d'une pluralité d'échafaudages comprenant au moins une fois le second échafaudage (21), occupe au moins 70 % d'un volume de la seconde partie (20).

7. Implant mammaire selon l'une quelconque des revendications précédentes, dans lequel la première partie (10) présente une extension radiale horizontale (19) qui est comprise entre 10 % et 65 % de l'extension horizontale (9) de la base (2).

8. Implant mammaire selon l'une quelconque des revendications précédentes, dans lequel la seconde partie (20) présente une densité inférieure à celle de la première partie (10).

9. Implant mammaire selon l'une quelconque des revendications précédentes, dans lequel un volume de chaque premier motif (12) est inférieur à un volume de chaque second motif (22).

10. Implant mammaire selon l'une quelconque des revendications précédentes, dans lequel le premier motif (12) présente des ouvertures (68) et le second motif (22) présente des ouvertures (78) telles que les ouvertures (68) du premier motif (12) sont plus petites que les ouvertures (78) du second motif (22).

11. Implant mammaire selon l'une quelconque des revendications précédentes, dans lequel les premiers motifs (12) présentent une dimension (17) comprise entre 1 mm et 8 mm.

12. Implant mammaire selon l'une quelconque des revendications précédentes, dans lequel les seconds motifs (22) présentent une dimension (27) comprise entre 2 mm et 10 mm.

13. Implant mammaire selon l'une quelconque des revendications précédentes, dans lequel l'implant mammaire est un assemblage de la première partie (10) et de la seconde partie (20).
